# EUROPEAN PATENT SPECIFICATION

(11) **EP 4 360 617 B1**
(45) Date of publication and mention of the grant of the patent: **02.07.2025**
(21) Application number: 23205451.0
(22) Date of filing: 24.10.2023
(51) Int. Cl.: A61K 8/41, A61Q 5/00, A61Q 5/12, A61Q 5/02

(54) **FORMULATION COMPRISING ESTER QUATS BASED ON TRIALKANOLAMINES**
FORMULIERUNG ENTHALTEND ESTERQUATS AUF BASIS VON TRIALKANOLAMINEN
FORMULATION COMPRENANT DES ESTERQUATS À BASE DE TRIALCANOLAMINES

(30) Priority: 31.10.2022 EP 22204643
(43) Date of publication of application: 01.05.2024
(73) Proprietor: Evonik Operations GmbH, 45128 Essen (DE)
(72) Inventor: Schwab, Peter, 45134 Essen (DE); Neubauer, Stefan, 50931 Köln (DE); Klann-Metz, Bärbel, 45141 Essen (DE)
(74) Representative: Evonik Patent Association

(56) References cited:
- EP-A1- 3 111 919
- EP-A2- 2 783 677
- EP-A2- 2 997 958
- US-A1- 2003 161 808

## Description

### Field of the invention

The invention provides compositions comprising specific ester quats based on trialkanolamines, and to the use of these compositions in cosmetics.

### Prior art

DE3608093 describes quaternary ammonium compounds comprising two 2-acyloxyalkyl groups, the acyl groups of which are derived from saturated or unsaturated carboxylic acids having 12 to 22 carbon atoms, including dimethyldi(oleoyl-oxyisopropyl)ammonium methosulfate, and the use of such substances in textile-softening formulations.

DE3877422 describes similar quaternary ammonium compounds, the corresponding acyl groups of which comprise at most 17 carbon atoms, including dimethyldi(palmitoyl-oxyisopropyl)ammonium chloride, and the use of such substances in textile-softening formulations, but also mentions their suitability for hair conditioning agents.

EP0900260 discloses a textile softener composition with improved stability and softening performance which comprises a fabric-softening active amount of a quaternary ammonium salt having specific mono-, di- and triester components, where the diester component amounts to more than about 55% by weight and the triester component amounts to less than about 25% by weight based on the total amount of the quaternary ammonium salt.

EP3820980A1 discloses a composition comprising, based on the weight of the composition, about 30% to about 100% by weight of a mixture of one or more ester quats and one or more glycerides, where the mixture comprises about 50% to about 80% by weight of ester quats and about 20% to about 50% by weight of glycerides; and, based on the weight of the composition, optionally 0% to about 70% by weight of a solvent.

US2003161808A1, EP2783677A2, EP3111919A1 and EP2997959, respectively, disclose ester quats or mixtures thereof which are structurally different to those presently claimed.

Ester quats obtainable hitherto, which are suitable for use in hair conditioning, are solid substances which are only converted to a formulatable form by using solvents, as a result of which it is stipulated which solvent will be present in the end formulation. This limits the degrees of freedom of the formulation options. Currently available liquid hair conditioning agents exhibit excellent conditioning properties but decrease the viscosity of the formulations as the concentration increases. This limits the use concentration and requires the use of an emulsifier for the fatty alcohol.

It was an object of the invention to provide a composition which exerts excellent conditioning effects on keratin fibres and simultaneously enables a high viscosity and stability.

### Description of the invention

Surprisingly, it has been found that the compositions described below are able to achieve the object of the invention.

The present invention provides a composition comprising an ester quat of the general formula I) 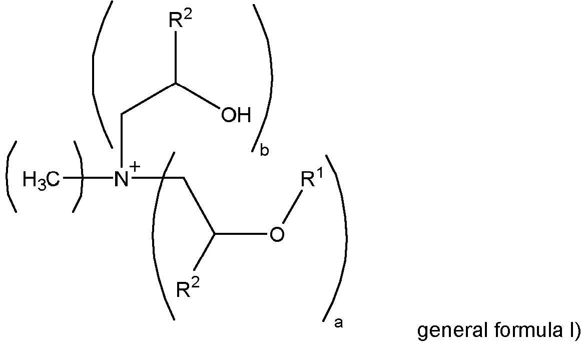 where
R¹ is an acyl radical of a fatty acid,
R² is independently selected from alkyl radicals having 1 to 6 carbon atoms and H, in particular H,
where
a = 1.0 to 2.2, preferably 1.5 to 2.0, particularly preferably 1.7 to 1.9, in particular 1.8, and
b = 3 - a,
characterized in that
R¹ is a mixture of acyl radicals which has
a weight ratio of saturated C16 acyl radicals to unsaturated C16 acyl radicals of greater than 2 and
a weight ratio of unsaturated C18 acyl radicals to saturated C18 acyl radicals of greater than 10.

It is clearly apparent to those skilled in the art from the general formula I) and the chosen wording that the ester quat of the composition according to the invention is a compound composed of multiple distinct molecules, with the numbers a to c stated indicating average values.

In the case where the mixture of acyl radicals does not comprise any unsaturated C16 acyl radicals or saturated C18 acyl radicals, then the respective ratio is greater than 2 or greater than 10.

The invention further provides for the use of a composition comprising an ester quat of the general formula I) 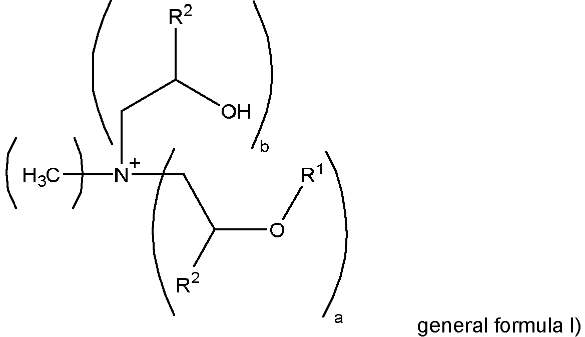 where
R¹ is an acyl radical of a fatty acid,
R² is independently selected from alkyl radicals having 1 to 6 carbon atoms and H, in particular H,
wherein
a = 1.0 to 2.2, preferably 1.5 to 2.0, particularly preferably 1.7 to 1.9, in particular 1.8, and
b = 3 - a,
characterized in that
R¹ is a mixture of acyl radicals which has
a weight ratio of saturated C16 acyl radicals to unsaturated C16 acyl radicals of greater than 2 and
a weight ratio of unsaturated C18 acyl radicals to saturated C18 acyl radicals of greater than 10, for the cosmetic treatment of keratin fibres, in particular hair, with preference being given to human hair.

One advantage of the present invention is that the ester quats used are liquid at room temperature and can therefore easily be incorporated into a final consumer formulation without the use of solvents and without heating, as a result of which such a solvent does not necessarily have to be present in said formulation.

Another advantage of the present invention is that the shine of the treated keratin fibres is increased.

A further advantage of the present invention is that the compounds used develop a good effect even in small use amounts.

A further advantage is that the compounds used have little impact from an ecological point of view. Another advantage is that the compounds used exhibit an improved conditioning effect on keratin fibres in the case of longer rinse-off times than quaternary ester compounds known hitherto. Another advantage of the present invention is that the compounds used do not crystallize out. Another advantage of the present invention is that the compounds used are effective in relatively low use concentrations.

Another advantage of the present invention is that the compounds used protect hair colourants from being washed out.

Another advantage of the present invention is that the compounds used protect keratin fibres against thermally induced damage.

Another advantage of the present invention is that the compounds used reduce the combing forces on wet and dry hair.

Another advantage of the present invention is that the compounds used are particularly economically viable.

Another advantage of the present invention is that the compounds used can be incorporated into surfactant formulations to give a clear formulation.

Another advantage of the present invention is that the viscosity can be increased depending on the use concentration of the conditioning agent.

Unless stated otherwise, all stated percentages (%) are percentages by mass.

The ester quats used in the context of the invention can be prepared by relevant methods of preparative organic chemistry. Usually, the preparation of ester quats is based on a multistage process in which firstly the esterified alkanolamine is prepared by reacting an alkanolamine with carboxylic acids or corresponding derivatives, and said alkanolamine is then subsequently quaternized with a suitable reagent. In connection with the present invention, the alkanolamine used is triethanolamine or triisopropanolamine or mixtures thereof, in particular triethanolamine.

In connection with suitable preparation processes, reference may be made to EP0483195, according to which trialkanolamine is partially esterified in the presence of hypophosphorous acid with fatty acids, air is passed through and then quaternization is carried out with dimethyl sulfate or ethylene oxide. The compounds listed therein serve as plasticizers for textiles. DE4308794 describes the preparation of solid ester quats by carrying out the quaternization of the triethanolamine esters in the presence of suitable dispersants. Overviews on this topic can be found for example under R. Puchta et al. in Tens. Surf. Det., 30, 186 (1993), M. Brock in Tens. Surf. Det., 30, 394 (1993), R. Lagerman et al. in J. Am. Chem. Soc., 71, 97 (1994) or under I. Shapiro in Cosm. Toil., 109, 77 (1994).

The ester quats present in the compositions according to the invention are characterized by the parameter a with respect to their degree of esterification. The ester quat thus comprises corresponding mono-, di- and/or triesters, which result in a statistical average corresponding to the degree of esterification.

Suitable methods for quantitative determination are HPLC and NMR, with particular emphasis being given to the methodology disclosed in *Characterization of quaternized triethanolamine esters (esterquats) by HPLC, HRCGC and NMR* by Wilkes, A. J. et al. World Surfactants Congress, 4th, Barcelona, June 3-7, 1996.

Compositions that are preferred according to the invention comprise an ester quat of the general formula I) that comprises, based on all ester quats of the general formula I) that are present, less than 20% by weight, preferably less than 15% by weight, especially preferably less than 10% by weight, of triester quat.

Compositions that are preferred according to the invention comprise an ester quat of the general formula I) in which R² = H, a = 1.7 to 1.9, in particular 1.8, and b = 3.

The acyl radicals R¹ of the general formula I) are a mixture which has a weight ratio of saturated C16 acyl radicals to unsaturated C16 acyl radicals of greater than 2 and a weight ratio of unsaturated C18 acyl radicals to saturated C18 acyl radicals of greater than 10.

Such a distribution can in particular be found in mixed vegetable oils.

Compositions that are preferred according to the invention are characterized in that the mixture of acyl radicals comprises a content of acyl radicals as shown below (mixed vegetable oil fatty acid I)

| | |
|---|---|
| C16 | 4% by weight - 7% by weight, |
| C16:1 | 0% by weight - 2% by weight, |
| C18 | 0% by weight - 4% by weight, |
| C18:1 | 55% by weight - 65% by weight, |
| C18:2 | 15% by weight - 25% by weight, |
| C18:3 | 6% by weight - 12% by weight, |

where the percentages by weight are based on the sum total of all R¹ in the ester quat of the general formula I). The iodine number is preferably in the range from 100 to 160, particularly preferably 105 to 135, gl/100 g.

Compositions that are preferred according to the invention are characterized in that the mixture of acyl radicals comprises a content of acyl radicals as shown below (mixed vegetable oil fatty acid II)

| | |
|---|---|
| C16 | 4% by weight - 7% by weight, |
| C16:1 | 0% by weight - 2% by weight, |
| C18 | 0% by weight - 4% by weight, |
| C18:1 | 14% by weight - 21% by weight, |
| C18:2 | 12% by weight - 25% by weight, |
| C18:3 | 29% by weight - 39% by weight, |

where the percentages by weight are based on the sum total of all R¹ in the ester quat of the general formula I). The iodine number is preferably in the range from 100 to 160, particularly preferably 105 to 135, gl/100 g.

Compositions that are preferred according to the invention are characterized in that the mixture of acyl radicals comprises a content of acyl radicals as shown below (tall oil fatty acid)

| | |
|---|---|
| C16 | 1% by weight - 8% by weight, |
| C16:1 | 0% by weight - 2% by weight, |
| C18 | 0% by weight - 4% by weight, |
| C18:1 | 40% by weight - 60% by weight, |
| C18:2 | 30% by weight - 40% by weight, |
| C18:3 | 5% by weight - 10% by weight, |

where the percentages by weight are based on the sum total of all R¹ in the ester quat of the general formula I). The iodine number is preferably in the range from 140 to 160 gl/100 g.

In the abovementioned compositions according to the invention, the mixture of acyl radicals may of course comprise a content of other acyl radicals in addition to those specifically named. It is preferable according to the invention if the sum total of the specifically named acyl radicals amounts to at least 70% by weight, in particular at least 80% by weight, particularly preferably at least 90% by weight, based on all acyl radicals present in the mixture.

Compositions that are preferred according to the invention are characterized in that the mixture of acyl radicals has an iodine number of 80 to 170, preferably 95 to 160, particularly preferably 105 to 135, cg I/g.

The charge of the compound I) present in the composition according to the invention must be compensated by corresponding anions; this takes place by means of counteranions present in the composition according to the invention.

Suitable as such counteranions are, for example, the halides, pseudohalides, anions of mineral acids, sulfates, sulfites, hydrogensulfites, sulfonate, alkyl- and arylsulfonates, phosphate, hydrogenphosphates, phosphites, hydrogenphosphites, phosphonites, carboxylates, borates, carbonates, sulfides, hydrogensulfides, lactate, glycolate, formate, acetate and propionate.

These anions are preferably selected from those which are suitable for cosmetic application and are therefore for example nontoxic. Particularly preferably, at least one counteranion to the compound of the general formula I) selected from the group comprising chloride, bromide, iodide, alkyl sulfate, for example methyl sulfate, ethyl sulfate, alkylsulfonate, for example methylsulfonate, triflate, tosylate, phosphate, sulfate, hydrogensulfate, lactate, glycolate, acetate and citrate, preferably chloride and methyl sulfate, is present.

The compositions according to the invention may be processed with further constituents to form formulations, in particular cosmetic formulations, so that the compositions according to the invention are, in particular cosmetic, formulations.

The formulations according to the invention comprise the ester quat preferably in an amount of 0.2% by weight to 25% by weight, preferably 0.5% by weight to 15% by weight, in particular 1% by weight to 10% by weight, where the percentages by weight are based on the total composition.

Formulations that are preferred according to the invention are characterized in that they do not comprise any fatty acids or fatty acid salts.

It has proven to be advantageous if the formulations according to the invention additionally comprise 0.5% by weight to 20% by weight, preferably 1.0% by weight to 10% by weight, in particular 2.0% by weight to 7.0% by weight, of at least one fatty alcohol, where the percentages by weight are based on the total formulation.

In this context, "fatty alcohol" is preferably understood to mean an unbranched or branched monoalcohol having an alkyl group of 8 to 30 carbon atoms, which may also be unsaturated. Preferred fatty alcohols are octanol, decanol, lauryl alcohol, isolauryl alcohol, anteisolauryl alcohol, myristyl alcohol, isomyristyl alcohol, cetyl alcohol, palmoleyl alcohol, stearyl alcohol, isostearyl alcohol, anteisostearyl alcohol, eicosanol, petroselinyl alcohol, Guerbet alcohol, arachyl alcohol, gadoleyl alcohol, behenyl alcohol, erucyl alcohol, hectacosanol, octacosanol, and melissyl alcohol, and mixtures thereof, in particular technical-grade mixtures, preferably technical-grade coconut or tallow fatty alcohols having 12 to 18, preferably having 16 to 18, carbon atoms, and the monounsaturated fatty alcohols, such as oleyl alcohol, elaidyl alcohol, delta-9-cis-hexadecenol, delta-9-octadecenol, trans-delta-9-octadecenol, cis-delta-11-octadecenol, trans-10,cis-12-hexadecadien-1-ol, octacosa-10,19-dien-1-ol, and polyunsaturated fatty alcohols such as for example linoleyl alcohol (9Z, 12Z-octadecadien-1-ol), elaidolinoleyl alcohol (9E, 12E-octadecadien-1-ol), linolenyl alcohol (9Z, 12Z, 15Z-octadecatrien-1-ol), elaidolinolenyl alcohol (9E, 12E, 15-E-octadecatrien-1-ol), with particular preference being given to mixtures of coconut or tallow fatty alcohols having 16 to 18 carbon atoms.

It is preferable according to the invention that the composition according to the invention comprises at least one alkylamidoamine.

Alkylamidoamines preferably present are those of the general formula II)

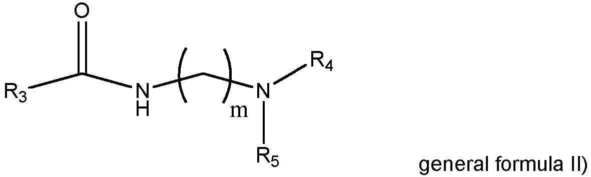

R₃CO here is an aliphatic, linear or branched acyl residue having 6 to 22 carbon atoms and 0 and/or 1, 2 or 3 double bonds, which generally originates from a naturally occurring or synthetically prepared fatty acid.

R₄ and R₅ are identical or different alkyl radicals which may optionally bear functional groups such as hydroxy groups, ester groups, amines, amides or other polar substituents, with preference however generally being given to unsubstituted hydrocarbon radicals which at most have one or more branches.

However, short-chain hydrocarbon radicals having 1 to 6 carbon atoms are especially preferred, with very particular preference being given according to the invention to ethyl and methyl radicals.

The number m is an integer from 1 to 10, with preference being given to values for m of 2 to 7, in particular of 2 to 4.

The alkylaminoamines are usually prepared by amidation of natural or synthetic fatty acids and fatty acid cuts with dialkylaminoamines. Typical examples of such fatty acids are caproic acid, caprylic acid, 2-ethylhexanoic acid, capric acid, lauric acid, isotridecanoic acid, myristic acid, palmitic acid, palm oleic acid, stearic acid, isostearic acid, oleic acid, elaidic acid, petroselinic acid, linoleic acid, linolenic acid, eleostearic acid, arachidic acid, gadoleic acid, behenic acid and erucic acid, and technical-grade mixtures thereof which are produced for example during the pressurized cleavage of natural fats and oils, during the reduction of aldehydes from the Roelen oxo synthesis or the dimerization of unsaturated fatty acids.

Particular preference is usually given to the fatty acid cuts which are obtainable from coconut oil or palm oil, with the use of stearic acid generally being especially preferred.

Depending on the intended use, the composition according to the invention may comprise the ester quat and the alkylamidoamine in different amounts.

In the treatment of keratin fibres, in particular of human hair, use is generally made of active ingredient concentrations (based on the individual components) of 0.2% by weight to 7% by weight, preferably 0.3% by weight to 5% by weight and especially preferably 0.4% by weight to 3% by weight. Particularly good results can be achieved at active ingredient concentrations of 0.2% to 2% by weight. The application of the preparations according to the invention to keratin fibres, in particular to human hair, is not however limited to the use of the active ingredients at a low concentration. It is also possible to use concentrates in which the active ingredients are each present in proportions of 2% by weight to 20% by weight or 3% by weight to 14% by weight, in particular at 5% by weight to 12% by weight.

Ester quats and alkylaminoamines are usually present in a weight ratio of 20 to 1 to 1 to 20, in particular of 10 to 1 to 1 to 10, in the preparation according to the invention. Depending on the desired effect, the mixing ratio may also be varied from 5 : 1 to 1 : 5 or even from 3 : 1 to 1 : 3.

The formulations according to the invention may comprise for example at least one further, additional component selected from the group of
emollients,
emulsifiers,
thickeners/viscosity regulators/stabilizers,
antioxidants,
hydrotropes (or polyols),
solids and fillers,
pearlescence additives,
deodorant and antiperspirant active ingredients,
insect repellents,
self-tanning agents,
preservatives,
conditioning agents,
perfumes,
colourants,
cosmetic active ingredients,
care additives,
superfatting agents,
solvents.

Substances which can be used as exemplary representatives of the individual groups are known to those skilled in the art and can be taken, for example, from German patent application DE 102008001788.4.

As regards further optional components and also the amounts used of these components, reference is expressly made to the relevant handbooks known to those skilled in the art, for example K. Schrader, "Grundlagen und Rezepturen der Kosmetika" [Fundamentals and formulations of cosmetics], 2nd edition, pages 329 to 341, Hüthig Buch Verlag, Heidelberg.

The amounts of the respective additives are guided by the intended use.

Typical starting formulations for the relevant applications are known prior art and are contained for example in the brochures of the manufacturers of the relevant base materials and active substances. These existing formulations can generally be adopted unchanged. However, if necessary, for adjustment and optimization, the desired modifications can be made in a straightforward manner through simple tests.

It is preferable according to the invention that the composition according to the invention comprises at least one emulsifier selected from the group of polyglycerol esters.

It is preferable according to the invention if the formulation has a pH of 3.0 to 5.5, preferably 3.5 to 5.0.

In connection with the present invention, the "pH" is defined as the value which is measured for the relevant composition at 25°C after stirring for five minutes using a pH electrode calibrated in accordance with ISO 4319 (1977).

The compositions according to the invention can be used according to the invention for treating keratin fibres, in particular for treating hair.

In this connection, use is preferably made of those compositions that are described above as preferred compositions.

The use according to the invention leads to the improvement in the conditioning, shine, flexibility, elasticity and/or combability, and to a reduction in the probability of breakage of the treated fibres and, moreover, it reduces the antistatic forces between the fibres.

The use according to the invention leads to the protection of the fibres against heat.

The examples which follow describe the present invention by way of example, without any intention of restricting the invention, the scope of application of which is apparent from the entirety of the description and the claims, to the embodiments cited in the examples.

The following figures are an integral part of the examples:
Fig. 1. Comparison of the reduction in the combing forces between formulations after a rinsing time of 1 minute

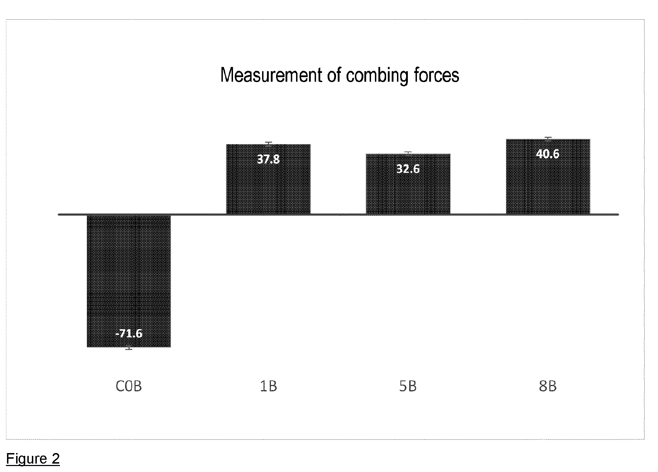

Fig. 2. Comparison of the reduction in the combing forces between formulations after a rinsing time of 3 minutes

### Examples:

### Example 1a: Preparation of "mixed vegetable oil fatty acid, reaction product with triethanolamine, quaternized with dimethyl sulfate" (inventive)

608 g (2.16 mol) of mixed vegetable oil fatty acid I (C16 4.7%, C16:1 0.2%, C18 1.6%, C18:1 60.2%, C18:2 18.7 %, C18:3 7.3%, iodine number 110 gl/100 g) is mixed with 182.4 g (1.22 mol) of triethanolamine and heated to 190°C with stirring. Water of reaction is distilled off continuously. After the majority of the water of reaction has been distilled at atmospheric pressure, reduced pressure is applied and the acid number of the reaction mixture is reacted down to < 6 mg KOH/g. The resulting ester amine is cooled to 60°C and admixed with 137.6 g (1.09 mol) of dimethyl sulfate in such a way that the reaction temperature does not exceed 90°C.

After cooling to room temperature, the total amine number (TAN) and the active content of the finished product are analysed.

TAN = 5.0 mg KOH/g; active content 1.16 meq/g (cationic active content according to Epton).

### Example 1b: Preparation of "mixed vegetable oil fatty acid, reaction product with triethanolamine, quaternized with dimethyl sulfate" (inventive)

563 g (2.0 mol) of mixed vegetable oil fatty acid II (C16 6.2%, C16:1 0.5%, C18 2.2%, C18:1 20.1%, C18:2 23.5%, C18:3 36.3%, iodine number 160 gl/100 g) is mixed with 168.5 g (1.13 mol) of triethanolamine and heated to 190°C with stirring. Water of reaction is distilled off continuously.

After the majority of the water of reaction has been distilled at atmospheric pressure, reduced pressure is applied and the acid number of the reaction mixture is reacted down to < 6 mg KOH/g. The resulting ester amine is cooled to 60°C and admixed with 128.3 g (1.02 mol) of dimethyl sulfate in such a way that the reaction temperature does not exceed 90°C.

After cooling to room temperature, the total amine number (TAN) and the active content of the finished product are analysed.

TAN = 4.9 mg KOH/g; active content 1.15 meq/g (cationic active content according to Epton).

### Example 2: Preparation of "stearic acid, reaction product with triethanolamine, quaternized with dimethyl sulfate" (non-inventive)

800 g (3.12 mol) of stearic acid (technical-grade quality, approx. 98% pure) is admixed with 263 g (1.76 mol) of triethanolamine and esterified as described in Example 1. The ester amine had an acid number of 5.6 mg KOH/g. This mixture was alkylated with 198 g (1.57 mol) of dimethyl sulfate as described in Example 1. At the end of the alkylation, the viscosity increased sharply and the mixture was diluted with 215 g of isopropanol.

The TAN of the finished product was determined with 4.0 mg KOH/g, the active content was 0.96 meq/g.

### Example 3: Preparation of "tall oil fatty acid, reaction product with triethanolamine, quaternized with dimethyl sulfate" (inventive)

814 g (2.84 mol) of commercial tall oil fatty acid (Sylfat^{™} 2 from Kraton, CAS No. 61790-12-3) is mixed with 240 g (1.60 mol) of triethanolamine and esterified as described in Example 1. The ester amine had an acid number of 4.5 mg KOH/g. This mixture was alkylated with 168 g (1.33 mol) of dimethyl sulfate as described in Example 1. The TAN of the finished product was determined with 6.3 mg KOH/g, the active content was 1.18 meq/g.

### Example 4: Preparation of "erucic acid, reaction product with triethanolamine, quaternized with dimethyl sulfate" (non-inventive)

392 g (1.15 mol) of erucic acid is mixed with 97 g (0.65 mol) of triethanolamine and esterified as described in Example 1. The ester amine had an acid number of 3.9 mg KOH/g. This mixture was alkylated with 73.7 g (0.59 mol) of dimethyl sulfate as described in Example 1. The TAN of the finished product was determined with 3.9 mg KOH/g, the active content was 1.13 meq/g.

### Example 5: Preparation of "isostearic acid, reaction product with triethanolamine, quaternized with dimethyl sulfate" (non-inventive)

504 g (1.77 mol) of isostearic acid (technical-grade quality) is admixed with 149.2 g (1.0 mol) of triethanolamine and esterified as described in Example 1. The ester amine had an acid number of 3.4 mg KOH/g. This mixture was alkylated with 113.5 g (0.9 mol) of dimethyl sulfate as described in Example 1.

The TAN of the finished product was determined with 5.1 mg KOH/g, the active content was 1.16 meq/g.

### Example 6: Preparation of "oleic acid, reaction product with triethanolamine, quaternized with dimethyl sulfate" (non-inventive)

500 g (1.77 mol) of oleic acid (98%) is admixed with 149.2 g (1.0 mol) of triethanolamine and esterified as described in Example 1. The ester amine had an acid number of 3.4 mg KOH/g. This mixture was alkylated with 113.5 g (0.9 mol) of dimethyl sulfate as described in Example 1.

The TAN of the finished product was determined with 5.5 mg KOH/g, the active content was 1.19 meq/g.

### Example 7: Preparation of "1-propanaminium, 2-hydroxy-N-(2-hydroxypropyl)-N,N-dimethyl, diester with mixed vegetable oil fatty acid, methyl sulfate" (non-inventive)

1120 g (4 mol) of mixed vegetable oil fatty acid is mixed with 302 g (2.05 mol) of methyldiisopropanolamine and heated to 180°C with stirring. Water of reaction is distilled off continuously. After the majority of the water of reaction has been distilled at atmospheric pressure, reduced pressure is applied and the acid number of the reaction mixture is reacted down to < 7 mg KOH/g. The resulting ester amine is cooled to 60°C and admixed in portions with 240 g (1.90 mol) of dimethyl sulfate in such a way that the reaction temperature does not exceed 100°C. After cooling to room temperature, the total amine number (TAN) and the active content of the finished product are analysed.

TAN = 5.0 mg KOH/g; active content 1.27 meq/g (cationic active content according to Epton).

### Example 8: Application technology of hair treatment agents using the examples and commercial market products.

For the application-related assessment, use was made of hair tresses which had been predamaged in a standardized manner by way of a bleaching treatment. Standard hairdressing products are used for this purpose. The damage to the hair tresses is described in detail in DE10327871.

For the application-related assessment, the compounds of the examples were used in a simple cosmetic formulation.

As reference compounds, the commercially available ester quat (INCI) Distearoylethyl Hydroxyethylmonium Methosulfate (VARISOFT^{®} EQ F 75 Pellets, Evonik Industries) was also used.

The application properties when used in hair rinses were tested in the following formulations (Tab. 1):

**Tab. 1: Hair rinse formulation for testing the hair conditioning properties**

| Formulation examples | C0a | 1a | V2a | 3a | V4a | V5a | V6a | V7a | 8a |
|---|---|---|---|---|---|---|---|---|---|
| TEGINACID^{®} C, Evonik Industries (INCI: Ceteareth-25) | 0.5% | 0.5% | 0.5% | 0.5% | 0.5% | 0.5% | 0.5% | 0.5% | 0.5% |
| TEGO^{®}Alkanol 1618, Evonik Industries (INCI: Cearyl Alcohol) | 5% | 5% | 5% | 5% | 5% | 5% | 5% | 5% | 5% |
| Example 1a (100%) (TEA mixed vegetable oil I) | | 1.5% | | | | | | | |
| Example 1b (100%) (TEA mixed vegetable oil II) | | | | | | | | | 1.5% |
| Example 2 (80% in isopropanol) | | | 1.88% | | | | | | |
| Example 3 (TEA tall oil) | | | | 1.5% | | | | | |
| Example 4 (TEA Eruca) | | | | | 1.5% | | | | |
| Example 6 (TEA oleic acid) | | | | | | 1.5% | | | |
| VARISOFT^{®} EQ F 75 Pellets (65% strength in C1618 fatty alcohol), Evonik Industries (INCI: Distearoylethyl Hydroxyethylmonium Methosulfate, Cetearyl Alcohol) | | | | | | | 2.3% | | |
| Example 7 | | | | | | | | 1.5% | |
| Water, demineralized | to 100.0 | | | | | | | | |
| Citric acid | to pH 4.0 | | | | | | | | |

The composition of the test formulations is deliberately chosen to be simple in order to avoid the test results being influenced by (normally present) formulation constituents. In addition to the specified ingredients and/or instead of the specified ingredients, formulations according to the invention may also comprise further ingredients. In particular, the combination with further ingredients can lead to a synergistic improvement in the case of the described effects.

The hair is pretreated using a shampoo formulation (Tab. 2) which does not comprise any conditioning agents.

**Tab. 2: Shampoo formulation for the pretreatment of the hair tresses.**

| | |
|---|---|
| Texapon NSO^{®}, 28% strength, Cognis (INCI: Sodium Laureth Sulfate) | 42.9% |
| NaCl | 3% |
| Water, demineralized | to 100.0 |

### Standardized treatment of predamaged hair tresses with conditioning samples:

The hair tresses which have been predamaged as described above are washed with the shampoo formulation from Tab. 2.

Here, the hair tresses are wetted under running warm water. The excess water is gently squeezed out by hand, then the shampoo is applied and worked gently into the hair for 1 min (0.5 ml/2 g hair tress). The hair tress is rinsed for 30 s under running warm water. This procedure is repeated once more, except that final rinsing is for 1 min.

Then, directly after washing, the hair tresses are conditioned with the hair rinse formulations from Tab. 1.

Here, the rinse is applied and gently worked into the hair (0.5 ml/2 g hair tress). After a residence time of 1 min, the hair is rinsed for a) 1 min or for b) 3 min.

Before the sensory assessment, the hair is dried for at least 12 h in the air at 50% humidity and 25°C.

### Assessment criteria:

The sensory evaluations are performed using grades awarded on a scale from 1 to 5, with 1 being the worst evaluation and 5 being the best evaluation. The individual test criteria each receive their own evaluation.

The test criteria are:
Wet combability, wet feel, dry combability, dry feel, appearance/shine.

In the tables which follow, the results of the sensory assessment of the treatment of the hair tresses, carried out as described above, in the case of a) 1 min rinsing time and in the case of b) 3 min rinsing time, are compared with the inventive formulations 1a and 8a and the comparative formulations and the control formulation C0a (control without test substance).

### a) 1 min rinsing time

**Tab. 3a: Results of the conditioning of hair with 1 min rinsing time**

| | Wet combability | Wet feel | Dry combability | Dry feel |
|---|---|---|---|---|
| Inventive formulation 1a | 5 | 5 | 4.5 | 4.5 |
| Comparative formulation V2a (TEA stearic acid) | 3 | 3 | 3 | 3 |
| Comparative formulation V5a (TEA oleic acid) | 4 | 4 | 4 | 4 |
| Comparative formulation V6a (EQ F 75) | 3 | 3 | 3 | 3 |
| Comparative formulation V7a (Ex. 7) | 4.5 | 4.5 | 4.5 | 4.5 |
| Inventive formulation 8a | 5 | 5 | 5 | 5 |
| Control C0a | 2 | 2 | 3 | 2.5 |

### b) 3 min rinsing time

**Tab. 3b: Results of the conditioning of hair with 3 min rinsing time**

| | Wet combability | Wet feel | Dry combability | Dry feel |
|---|---|---|---|---|
| Inventive formulation 1a | 4.5 | 4.5 | 4.5 | 4.5 |
| Comparative formulation V2a (TEA stearic acid) | 2.5 | 2.5 | 2.5 | 3.5 |
| Comparative formulation V5a (TEA oleic acid) | 3.5 | 3.5 | 3.5 | 3.5 |
| Comparative formulation V6a (EQ F 75) | 2.5 | 3 | 3 | 3 |
| Comparative formulation V7a (Ex. 7) | 4.5 | 4.5 | 4 | 4 |
| Inventive formulation 8a | 5 | 5 | 5 | 5 |
| Control C0a | 2 | 2 | 3 | 2.5 |

The results in Table 3a show that the inventive formulations 1a and 8a have very good conditioning properties with 1 min rinsing time which are at the same level as the comparative formulation V7a and are very significantly superior to the comparative formulations V2a and V6a. The inventive formulations 1a and 8a are noticeably improved in comparison with V5a.

The results in Table 3b show that the inventive formulations 1a and 8a have even more markedly improved conditioning properties with 3 min rinsing time than after 1 min than the comparative formulations V2a and V6a. In comparison with the comparative formulation V7a, the results are comparable at a very high level. The comparative formulation V7a comprises an ester quat that is known for its very good conditioning properties even in the case of long rinsing times. The comparative formulation V6a comprises, as conditioning compound, VARISOFT^{®} EQ F 75 (65% strength in C16/18 fatty alcohol, Evonik Industries, INCI: Distearoylethyl Dimonium Chloride, Cetearyl Alcohol), an ester quat that has very good emulsifying properties, but only a moderate conditioning performance with 1 min rinsing time (see Tab. 3a), and exhibits even worse conditioning with 3 min rinsing time.

### Example 8: Influence of the compounds according to the invention on combing forces of hair

### Experimental conditions:

Measuring device: Diastron MTT 175
Measurement distance: 20 cm
Combing rate: 2000 mm/min
Hair tresses used: length = 23 cm; width = 1.5 cm; weight = 2 g Measurement conditions: T = 22°C The hair tresses are measured with a residual moisture of 60%, determined by weight determination. European, undamaged, dark brown hair is used for the experiments. To carry out the combing force measurements, this hair is damaged by means of perming in the laboratory in accordance with standard conditions:
   1.) 4 g of perming solution/g of hair, leave to act for 15 min, rinse out for 2 min under running tap water (T = 35°C). (Perming solution: Universal perming, Basler) 2.) 4 g of neutralizer (1 part neutralizing solution + 3 parts water)/g of hair, leave to act for 10 min, rinse out for 2 min. (Neutralizing solution: foam neutralizer concentrate, Basler)
   Carrying out the combing force measurement before the treatment with the test formulation:
      The predamaged hair tresses are climatized overnight.
      3.) The hair tress is dipped for 1 min in a buffer solution (Na citrate, pH = 6). 4.) The hair tress is precombed by hand until no change in combing resistance can be ascertained. 5.) The hair tress is clamped in the measuring device and the first combing force measurement is carried out. The measurement is repeated a total of 10 times.

### Treatment of the tresses:

0.5 g of the respective test formulation is used per hair tress (2 g hair/0.5 g solution). The formulation is massaged into the hair for 30 sec and then left on for 5 min, then rinsed off under running tap water for 1 min or 3 min.

Carrying out the combing force measurement after the treatment with the test formulation:
Points 3-5 are repeated.

The combability (%) is then calculated before and after the treatment with the test formulation. Test formulations used:
The combing forces when used in hair rinses were tested in the following formulations (Tab. 4):

**Tab. 4: Hair rinse formulations for testing the hair conditioning properties**

| Formulation examples | C0b | 1b | V2b | 3b | V4b | V5b | V6b | V7b | 8b |
|---|---|---|---|---|---|---|---|---|---|
| TEGINACID^{®} C, Evonik Industries (INCI: Ceteareth-25) | 0.5% | 0.5% | 0.5% | 0.5% | 0.5% | 0.5% | 0.5% | 0.5% | 0.5% |
| TEGO^{®}Alkanol 1618, Evonik Industries (INCI: Cearyl Alcohol) | 5% | 5% | 5% | 5% | 5% | 5% | 5% | 5% | 5% |
| Example 1a (100%) (TEA mixed vegetable oil I) | | 1.5% | | | | | | | |
| Example 1b (100%) (TEA mixed vegetable oil II) | | | | | | | | | 1.5% |
| Example 2 (80% in isopropanol) | | | 1.88% | | | | | | |
| Example 3 (TEA tall oil) | | | | 1.5% | | | | | |
| Example 4 (TEA Eruca) | | | | | 1.5% | | | | |
| Example 6 (TEA oleic acid) | | | | | | 1.5% | | | |
| VARISOFT^{®} EQ 75 Pellets (65% strength in C1618 fatty alcohol), Evonik Industries (INCI: Distearoylethyl Hydroxyethylmonium Methosulfate, Cetearyl Alcohol) | | | | | | | 2.3% | | |
| Example 7 | | | | | | | | 1.5% | |
| Water, demineralized | to 100.0 | | | | | | | | |
| Citric acid | to pH 4.0 | | | | | | | | |

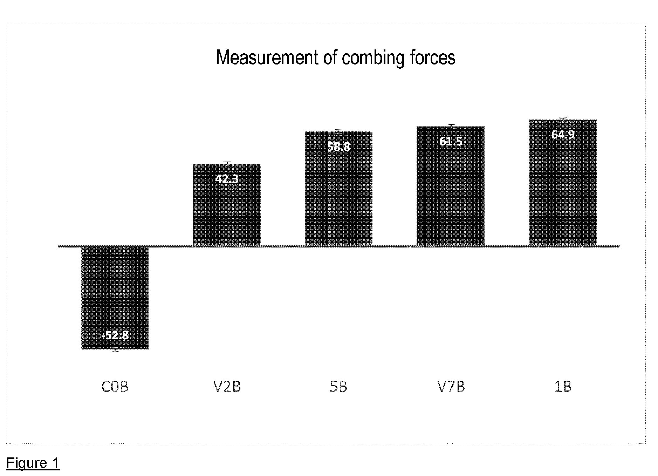

The results are shown in Figures 1 (rinsing time of 1 min) and 2 (rinsing time of 3 min).

### Example 9: Antistatic finishing of keratin fibres

To test the antistatic behaviour, the shadow silhouette method was used.

The pretreated hair tresses described above, a plastic comb, a spotlight and a projection field marked with concentric semicircles are used.

The experiments were carried out under standardized climatic conditions. The hair tress is hung up at a distance of 15 cm from the projection field. The spotlight is positioned at a distance of 145 cm from the hair tress so that a shadow falls on the projection field.

The hair tress is then combed five times in succession using the comb. The electrostatic charging is measured via the shadow silhouette by marking the two outer points of the shadow and determining the distance between them. The smaller the shadow area, the more effective the antistatic effect.

### Result:

| Formulation | Distance |
|---|---|
| C0a | 15 cm |
| 1a | 4 cm |
| V7a | 6 cm |

### Example 10: Viscosities of the hair rinse formulations

| Formulation | Viscosity after preparation | Viscosity after storage at RT (1 month) | Viscosity after storage at RT (6 months) | Viscosity after storage at 40°C (3 months) |
|---|---|---|---|---|
| Inventive formulation 1a | 15 Pas | 15 Pas | 15 Pas | 10 Pas |
| Comparative formulation 2a (TEA stearic acid) | 12 Pas | 12 Pas | 12 Pas | 5 Pas |
| Comparative formulation 6a (EQ F 75) | 11.5 Pas | 11.5 Pas | 11 Pas | 5 Pas |
| Comparative formulation 7a (Ex. 7) | 7 Pas | 6 Pas | Phase separation | Phase separation |
| Comparative formulation 5a (TEA oleic acid) | 13 Pas | 13 Pas | 12 Pas | 6 Pas |

### Example formulations

Each individual formulation listed below was prepared three times.

For "Inventive Example X", each of Inventive Examples 1a, 1b and 3 were used.

### Formulation Example 1) Pearlized shampoo

| | |
|---|---|
| TEXAPON^{®} NSO, Cognis, 28% strength (INCI: Sodium Laureth Sulfate) | 32.25% |
| Inventive Example X | 0.25% |
| Perfume | 0.25% |
| Water | 55.25% |
| TEGO^{®}Betain F 50, Evonik Industries AG, 38% strength (INCI: Cocamidopropyl Betaine) | 8.00% |
| TEGO^{®}Pearl N 300 Evonik Industries AG (INCI: Glycol Distearate; Laureth-4; Cocamidopropyl Betaine) | 2.00% |
| ANTIL^{®} 171 Evonik Industries AG (INCI: PEG-18 Glyceryl Oleate/Cocoate) | 1.50% |
| NaCl | 0.50% |
| Preservative | q.s. |

### Formulation Example 2) Rinse-Off Conditioner

| | |
|---|---|
| Water | 92.0% |
| TEGINACID^{®} C, Evonik Industries AG (INCI: Ceteareth-25) | 0.5% |
| Inventive Example X | 2.50% |
| TEGO^{®}Alkanol 1618, Evonik Industries AG (INCI: Cetearyl Alcohol) | 5.00% |
| Preservative, Perfume | q.s. |

### Formulation Example 3) Rinse-Off Conditioner

| | |
|---|---|
| Water | 91.0% |
| Inventive Example X | 2.00% |
| VARISOFT^{®} BT 85, Evonik Industries AG (INCI: Behentrimonium Chloride) | 2.00% |
| TEGO^{®}Alkanol 1618, Evonik Industries AG (INCI: Cetearyl Alcohol) | 5.00% |
| Preservative, Perfume | q.s. |

### Formulation Example 4) Rinse-Off Conditioner

| | |
|---|---|
| Water | 90.20% |
| Inventive Example X | 2.00% |
| VARISOFT^{®} EQ 65, Evonik Industries AG (INCI: Distearoyl Dimonium Chloride, Cetearyl Alcohol) | 2.00% |
| TEGO^{®}Alkanol 1618, Evonik Industries AG (INCI: Cetearyl Alcohol) | 5.80% |
| Preservative, Perfume | q.s. |

### Formulation Example 5) Rinse-Off Conditioner

| | |
|---|---|
| Water | 89.20% |
| TEGINACID^{®} C, Evonik Industries AG (INCI: Ceteareth-25) | 0.5% |
| VARISOFT^{®} EQ 65, Evonik Industries AG (INCI: Distearoyl Dimonium Chloride, Cetearyl Alcohol) | 2.00% |
| Inventive Example X | 2.00% |
| ABIL^{®} Quat 3272, Evonik Industries AG (INCI: Quaternium-80) | 1.30% |
| TEGO^{®}Alkanol 1618, Evonik Industries AG (INCI: Cetearyl Alcohol) | 5.00% |
| Preservative, Perfume | q.s. |

### Formulation Example 6) Rinse-Off Conditioner

| | |
|---|---|
| TEGINACID^{®} C, Evonik Industries AG (INCI: Ceteareth-25) | 0.50% |
| TEGO^{®}Alkanol 16, Evonik Industries AG (INCI: Cetyl Alcohol) | 2.00% |
| TEGO^{®} Amid S 18, Evonik Industries AG (INCI: Stearamidopropyl Dimethylamine) | 1.00% |
| Inventive Example X | 1.50% |
| Propylene Glycol | 2.00% |
| Citric Acid Monohydrate | 0.30% |
| Water | 92.70% |
| Preservative, Perfume | q.s. |

### Formulation Example 7) Rinse-Off Conditioner

| | |
|---|---|
| TEGINACID^{®} C, Evonik Industries AG (INCI: Ceteareth-25) | 0.50% |
| TEGO^{®}Alkanol 16, Evonik Industries AG (INCI: Cetyl Alcohol) | 5.00% |
| TEGOSOFT^{®} DEC, Evonik Industries AG (INCI: Diethylhexyl Carbonate) | 1.00% |
| Inventive Example X | 1.50% |
| Water | 89.20% |
| TEGO^{®} Cosmo C 100 Evonik Industries AG (INCI: Creatine) | 0.50% |
| Propylene Glycol | 2.00% |
| Citric Acid Monohydrate | 0.30% |
| Preservative, Perfume | q.s. |

### Formulation Example 8) Leave-In Conditioner Spray

| | |
|---|---|
| Lactic Acid, 80% | 0.40% |
| Water | 95.30% |
| Inventive Example X | 1.20% |
| TEGIN^{®} G 1100 Pellets, Evonik Industries AG (INCI: Glycol Distearate) | 0.60% |
| TEGO^{®} Care PS, Evonik Industries AG (INCI: Methyl Glucose Sesquistearate) | 1.20% |
| TEGOSOFT^{®} DEC, Evonik Industries AG (INCI: Diethylhexyl Carbonate) | 1.30% |
| Preservative, Perfume | q.s. |

### Formulation Example 9) Leave-In Conditioner Spray

| | |
|---|---|
| Lactic Acid, 80% | 0.40% |
| Water | 95.30% |
| TEGO^{®} Amid S 18, Evonik Industries AG (INCI: Stearamidopropyl Dimethylamine) | 1.20% |
| Inventive Example X | 0.30% |
| TEGIN^{®} G 1100 Pellets, Evonik Industries AG (INCI: Glycol Distearate) | 0.90% |
| TEGO^{®} Care PS, Evonik Industries AG (INCI: Methyl Glucose Sesquistearate) | 1.60% |
| TEGOSOFT^{®} DEC, Evonik Industries AG (INCI: Diethylhexyl Carbonate) | 0.30% |
| Preservative, Perfume | q.s. |

### Formulation Example 10) Leave-In Conditioner Spray

| | |
|---|---|
| TAGAT^{®} CH-40, Evonik Industries AG (INCI: PEG-40 Hydrogenated Castor Oil) | 2.20% |
| Ceramide VI, Evonik Industries AG (INCI: Ceramide 6 II) | 0.05% |
| Perfume | 0.20% |
| Water | 90.95% |
| Inventive Example X | 0.30% |
| LACTIL^{®} Evonik Industries AG (INCI: Sodium Lactate; Sodium PCA; Glycine; Fructose; Urea; Niacinamide; Inositol; Sodium Benzoate; Lactic Acid) | 2.00% |
| TEGO^{®}Betain F 50 Evonik Industries AG 38% (INCI: Cocamidopropyl Betaine) | 2.30% |
| Citric Acid (10% in water) | 2.00% |

### Formulation Example 11) Leave-in Conditioner Foam

| | |
|---|---|
| Inventive Example X | 0.30% |
| TAGAT^{®} CH-40, Evonik Industries AG (INCI: PEG-40 Hydrogenated Castor Oil) | 1.0% |
| Perfume | 0.30% |
| TEGO^{®}Betain 810, Evonik Industries AG (INCI: Capryl/Capramidopropyl Betaine) | 2.00% |
| Water | 94.00% |
| TEGO^{®} Cosmo C 100, Evonik Industries AG (INCI: Creatine) | 0.50% |
| TEGOCEL^{®} HPM 50, Evonik Industries AG (INCI: Hydroxypropyl Methylcellulose) | 0.30% |
| VARISOFT^{®} 300, Evonik Industries AG (INCI: Cetrimonium Chloride) | 1.0% |
| LACTIL^{®} Evonik Industries AG (INCI: Sodium Lactate; Sodium PCA; Glycine; Fructose; Urea; Niacinamide; Inositol; Sodium Benzoate; Lactic Acid) | 0.50% |
| Citric Acid, 30% | 0.10% |
| Preservative | q.s. |

### Formulation Example 12) Strong Hold Styling Gel

| | |
|---|---|
| TEGO^{®}Carbomer 141, Evonik Industries AG (INCI: Carbomer) | 1.20% |
| Water | 66.70% |
| NaOH, 25% | 2.70% |
| PVP/VA W-735, ISP (INCI: PVP/VA Copolymer) | 16.00% |
| Inventive Example X | 0.30% |
| Alcohol Denat. | 10.50% |
| TAGAT^{®} O 2 V, Evonik Industries AG (INCI: PEG-20 Glyceryl Oleate) | 2.00% |
| Perfume | 0.30% |
| ABIL^{®} B 88183, Evonik Industries AG (INCI: PEG/PPG-20/6 Dimethicone) | 0.30% |
| Preservative | q.s. |

### Formulation Example 13) Body Care Composition

| | |
|---|---|
| TEXAPON^{®} NSO, Cognis, 28% strength (INCI: Sodium Laureth Sulfate) | 30.00% |
| TEGOSOFT^{®} PC 31, Evonik Industries AG (INCI: Polyglyceryl-3 Caprate) | 0.70% |
| Inventive Example X | 0.30% |
| Perfume | 0.30% |
| Water | 53.90% |
| TEGOCEL^{®} HPM 4000, Evonik Industries AG (INCI: Hydroxypropyl Methylcellulose) | 0.30% |
| REWOTERIC^{®} AM C, Evonik Industries AG, 32% strength (INCI: Sodium Cocoamphoacetate) | 10.00% |
| Citric Acid Monohydrate | 0.50% |
| REWODERM^{®} LI S 80, Evonik Industries AG (INCI: PEG-200 Hydrogenated Glyceryl Palmate; PEG-7 Glyceryl Cocoate) | 2.00% |
| TEGO^{®}Pearl N 300, Evonik Industries AG (INCI: Glycol Distearate; Laureth-4; Cocamidopropyl Betaine) | 2.00% |

### Formulation Example 14) Mild Foam Bath

| | |
|---|---|
| TEXAPON^{®} NSO, Cognis, 28% strength (INCI: Sodium Laureth Sulfate) | 27.00% |
| REWOPOL^{®} SB FA 30, Evonik Industries AG, 40% strength (INCI: Disodium Laureth Sulfosuccinate) | 12.00% |
| TEGOSOFT^{®} LSE 65 K SOFT, Evonik Industries AG (INCI: Sucrose Cocoate) | 2.00% |
| Water | 39.00% |
| REWOTERIC^{®} AM C, Evonik Industries AG, 32% strength (INCI: Sodium Cocoamphoacetate) | 13.00% |
| Inventive Example X | 0.40% |
| Citric Acid (30% in water) | 3.00% |
| ANTIL^{®} 171 Evonik Industries AG (INCI: PEG-18 Glyceryl Oleate/Cocoate) | 1.60% |
| TEGO^{®}Pearl N 300 Evonik Industries AG (INCI: Glycol Distearate; Laureth-4; Cocamidopropyl Betaine) | 2.00% |

### Formulation Example 15) Rinse-Off Conditioner

| | |
|---|---|
| Water | 89.20% |
| Inventive Example X | 3.00% |
| ABIL^{®} OSW 5, Evonik Industries AG (INCI: Cyclopentasiloxane; Dimethiconol) | 1.80% |
| TEGO^{®}Alkanol 1618, Evonik Industries AG (INCI: Cetearyl Alcohol) | 6.00% |
| Preservative, Perfume | q.s. |

### Formulation Example 16) Rinse-Off Conditioner

| | |
|---|---|
| Water | 89.20% |
| TEGINACID^{®} C, Evonik Industries AG (INCI: Ceteareth-25) | 0.5% |
| VARISOFT^{®} EQ 65, Evonik Industries AG (INCI: Distearyl Dimonium Chloride, Cetearyl Alcohol) | 1.50% |
| Inventive Example X | 2.00% |
| ABIL^{®} Soft AF 100, Evonik Industries AG (INCI: Methoxy PEG/PPG-7/3 Aminopropyl Dimethicone) | 1.00% |
| TEGO^{®}Alkanol 1618, Evonik Industries AG (INCI: Cetearyl Alcohol) | 5.80% |
| Preservative, Perfume | q.s. |

### Formulation Example 17) Rinse-Off Conditioner

| | |
|---|---|
| Water | 91.50% |
| TEGINACID^{®} C, Evonik Industries AG (INCI: Ceteareth-25) | 0.5% |
| Inventive Example X | 2.00% |
| SF 1708, Momentive (INCI: Amodimethicone) | 1.00% |
| TEGO^{®}Alkanol 1618, Evonik Industries AG (INCI: Cetearyl Alcohol) | 5.00% |
| Preservative, Perfume | q.s. |

### Formulation Example 18) 2in1 Shampoo & Intensive Conditioner

| | |
|---|---|
| Water | 28.00% |
| Jaguar C-162 (Hydroxypropyl Guar Hydroxypropyltimonium Chloride) | 0.5% |
| VARISOFT^{®}BT 85, Evonik Industries AG (INCI: Behentrimonium Chloride) | 0.50% |
| Inventive Example X | 0.50% |
| TEGO^{®}Alkanol 1618, Evonik Industries AG (INCI: Cetearyl Alcohol) | 1.00% |
| TEGIN^{®} G 1100 Pellets, Evonik Industries AG (INCI: Glycol Distearate) | 0.80% |
| Sodium Laureth Sulfate, 28% | 39.0% |
| Petrolatum | 1.0% |
| ABIL^{®}T- Quat 60, Evonik Industries AG (INCI: Silicone Quaternium-22) | 1.3% |
| REWOTERIC^{®} AM C, Evonik Industries AG (INCI: Sodium Cocoamphoacetate) | 17.50% |
| Citric Acid (20%) | 6.3% |
| ANTIL^{®} SPA 80, Evonik Industries AG (INCI: Isostearamide MIPA; Glyceryl Laurate) | 2.20% |
| NaCl | 1.0% |
| REWODERM^{®} LI S 80, Evonik Industries AG (INCI: PEG-200 Hydrogenated Glyceryl Palmate; PEG-7 Glyceryl Cocoate) | 1.0% |
| Preservative, Perfume | q.s. |

### Formulation Example 19: Pet Care - Conditioner

| | |
|---|---|
| TEGO^{®}Alkanol 1618, Evonik Industries AG (INCI: Cetearyl Alcohol) | 4.0% |
| TEGINACID^{®} C, Evonik Industries AG (INCI: Ceteareth-25) | 0.5% |
| VARISOFT^{®}BT 85, Evonik Industries AG (INCI: Behentrimonium Chloride) | 2.0% |
| Inventive Example X | 1.0% |
| TEGIN^{®} M, Evonik Industries AG (INCI: Glyceryl Stearate) | 0.5% |
| Water | 93.0% |
| Preservative, Perfume | q.s. |

### Formulation Example 20: In-Shower Hair and Body Conditioner (O/W emulsion)

| | |
|---|---|
| ABIL^{®} Soft AF 100, Evonik Industries AG (INCI: Methoxy PEG/PPG-7/3 Aminopropyl Dimethicone) | 0.5% |
| Inventive Example X | 1.0% |
| VARISOFT^{®}BT 85, Evonik Industries AG (INCI: Behentrimonium Chloride) | 0.5% |
| TEGO^{®}Alkanol 16, Evonik Industries AG (INCI: Cetyl Alcohol) | 2.0% |
| Simmondsia Chinensis (Jojoba) Seed Oil | 0.5% |
| Water | 93.5% |
| Glycerin | 2.0% |
| Panthenol | 0.2% |
| Preservative, Perfume | q.s. |

### Formulation Example 21: Hot Oil Treatment

| | |
|---|---|
| Water | 96.5 |
| Polyquaternium-10 | 1.0% |
| Hydroxyethylcellulose | 0.5% |
| Inventive Example X | 1.0% |
| VARISOFT^{®}BT 85, Evonik Industries AG (INCI: Behentrimonium Chloride) | 1.0% |
| Lauramide DEA | 1.0% |
| Citric Acid, 30% | q.s. |
| Preservative, Perfume | q.s. |

### Formulation Example 22: Strong conditioning hair rinse for damaged hair

| | |
|---|---|
| VARISOFT^{®}BT 85, Evonik Industries AG (INCI: Behentrimonium Chloride) | 0.5% |
| Inventive Example X | 0.5% |
| COSMOFERM^{®}Mix III, Evonik Industries AG (INCI: Isocetyl Alcohol; Ceramide NP; Cetyl Alcohol) | 2.0% |
| TEGINACID^{®} C, Evonik Industries AG (INCI: Ceteareth-25) | 0.5% |
| ABIL^{®} Soft AF 100, Evonik Industries AG (INCI: Methoxy PEG/PPG-7/3 Aminopropyl Dimethicone) | 0.3% |
| TEGO^{®}Alkanol 16, Evonik Industries AG (INCI: Cetyl Alcohol) | 2.0% |
| TEGIN^{®} M, Evonik Industries AG (INCI: Glyceryl Stearate) | 1.0% |
| ABIL^{®} OSW 5, Evonik Industries AG (INCI: Cyclopentasiloxane; Dimethiconol) | 7.5% |
| Water | 86.2% |
| Preservative, Perfume | q.s. |

### Formulation Example 23: Conditioning hair rinse for coloured hair

| | |
|---|---|
| TEGIN^{®} M, Evonik Industries AG (INCI: Glyceryl Stearate) | 2.0% |
| TEGO^{®}Alkanol 1618, Evonik Industries AG (INCI: Cetearyl Alcohol) | 3.0% |
| TEGO^{®}Amid S 18, Evonik Industries AG (INCI: Stearamidopropyl Dimethylamine) | 0.7% |
| VARISOFT^{®}BT 85, Evonik Industries AG (INCI: Behentrimonium Chloride) | 0.8% |
| Inventive Example X | 0.8% |
| VARISOFT^{®}W 575 PG, Evonik Industries AG (INCI: Quaternium-87) | 0.9% |
| Water | 90.9% |
| 1,2-Propylene glycol (Propylene Glycol) | 1.0% |
| Citric Acid (20%) | 0.7% |
| Preservative, Perfume | q.s. |

### Formulation Example 24: Creamy Conditioning Shampoo

| | |
|---|---|
| Plantacare^{®} 1200 UP, BASF SE (INCI: Lauryl Glucoside) | 8.8% |
| ANTIL^{®} SPA 80, Evonik Industries AG (INCI: Isostearamide MIPA; Glyceryl Laurate) | 1.0% |
| RHEANCE^{®} One, Evonik Industries AG (INCI: Glycolipids) | 0.7% |
| Jaguar^{®} C-162, Solvay (INCI: Hydroxypropyl Guar Hydroxypropyltrimonium Chloride) | 0.5% |
| Inventive Example X | 0.5% |
| REWOTERIC^{®} AM C MB, Evonik Industries AG (INCI: Sodium Cocoamphoacetate) | 12.5% |
| Water | Ad 100.0% |
| TEGO^{®} Betain P 50 C, Evonik Industries AG (INCI: (Cocamidopropyl Betaine) | 14.9% |
| Natrium Chloride (20%) | 0.8% |
| TEGO^{®} White 50 MB, Evonik Industries AG (INCI: Glycol Distearate; Disodium Lauryl Sulfosuccinate; Glyceryl Laurate) | 3.0% |
| Verstatil^{®} SL non GMO, Evonik Industries AG (INCI: Aqua; Sodium Levulinate; Potassium Sorbate) | 1.1% |

## Claims

1. Composition comprising an ester quat of the general formula I) 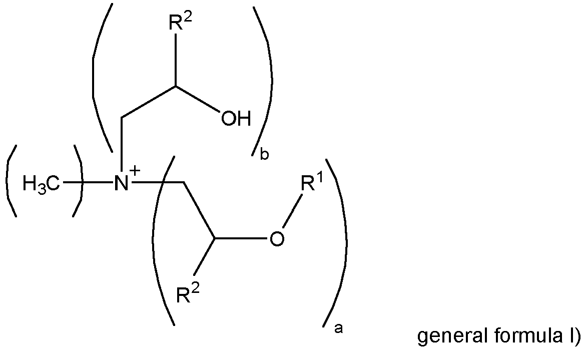 where
R¹ is an acyl radical of a fatty acid,
R² is independently selected from alkyl radicals having 1 to 6 carbon atoms and H, in particular H,
where
a = 1.0 to 2.2, preferably 1.5 to 2.0, particularly preferably 1.7 to 1.9, in particular 1.8, and
b = 3 - a,
**characterized in that**
R¹ is a mixture of acyl radicals which has
a weight ratio of saturated C16 acyl radicals to unsaturated C16 acyl radicals of greater than 2 and
a weight ratio of unsaturated C18 acyl radicals to saturated C18 acyl radicals of greater than 10.

2. Composition according to Claim 1, **characterized in that** the mixture of acyl radicals comprises a content of acyl radicals as shown below
| | |
|---|---|
| C16 | 4% by weight - 7% by weight, |
| C16:1 | 0% by weight - 2% by weight, |
| C18 | 0% by weight - 4% by weight, |
| C18:1 | 55% by weight - 65% by weight, |
| C18:2 | 15% by weight - 25% by weight, |
| C18:3 | 6% by weight - 12% by weight, |
where the percentages by weight are based on the sum total of all R¹ in the ester quat of the general formula I).

3. Composition according to Claim 1, **characterized in that** the mixture of acyl radicals comprises a content of acyl radicals as shown below
| | |
|---|---|
| C16 | 4% by weight - 7% by weight, |
| C16:1 | 0% by weight - 2% by weight, |
| C18 | 0% by weight - 4% by weight, |
| C18:1 | 14% by weight - 21% by weight, |
| C18:2 | 12% by weight - 25% by weight, |
| C18:3 | 29% by weight - 39% by weight, |
where the percentages by weight are based on the sum total of all R¹ in the ester quat of the general formula I).

4. Composition according to Claim 1, **characterized in that** the mixture of acyl radicals comprises a content of acyl radicals as shown below
| | |
|---|---|
| C16 | 1% by weight - 8% by weight, |
| C16:1 | 0% by weight - 2% by weight, |
| C18 | 0% by weight - 4% by weight, |
| C18:1 | 40% by weight - 60% by weight, |
| C18:2 | 30% by weight - 40% by weight, |
| C18:3 | 5% by weight - 10% by weight, |
where the percentages by weight are based on the sum total of all R¹ in the ester quat of the general formula I).

5. Composition according to any of Claims 2 to 4, **characterized in that** the sum total of the specifically named acyl radicals amounts to at least 70% by weight, in particular at least 80% by weight, particularly preferably at least 90% by weight, based on all acyl radicals present in the mixture.

6. Composition according to at least one of the preceding claims, **characterized in that** the mixture of acyl radicals has an iodine number of 80 to 170, preferably 95 to 160, particularly preferably 105 to 135, cg I/g.

7. Composition according to at least one of the preceding claims, additionally comprising at least one counteranion to the compound of the general formula I) selected from the group comprising chloride, bromide, iodide, alkyl sulfate, for example methyl sulfate, ethyl sulfate, alkylsulfonate, for example methylsulfonate, triflate, tosylate, phosphate, sulfate, hydrogensulfate, lactate, glycolate, acetate and citrate.

8. Composition according to at least one of the preceding claims, **characterized in that** the composition is a formulation, in particular a cosmetic formulation.

9. Composition according to at least one of the preceding claims, **characterized in that** the ester quat is present in an amount of 0.2% to 25% by weight, preferably 0.5% to 15% by weight, in particular 1% to 10% by weight, where the percentages by weight are based on the total composition.

10. Composition according to at least one of the preceding claims, **characterized in that** it comprises at least one alkylamidoamine, which is preferably present in an amount of 0.2% by weight to 25% by weight, preferably 0.5% by weight to 15% by weight, in particular 1.0% by weight to 10% by weight, where the percentages by weight are based on the total composition.

11. Use of a composition according to at least one of the preceding claims for the cosmetic treatment of keratin fibres.

12. Use according to Claim 11 for increasing the shine of the fibre.

13. Use according to Claim 11 for increasing the flexibility, the combability and/or the elasticity of the fibre.

14. Use according to Claim 11 for reducing the antistatic forces between the fibres.

15. Use according to Claim 11 for protecting the fibres against heat.

## Patentansprüche

1. Zusammensetzung enthaltend ein Esterquat der allgemeinen Formel I) 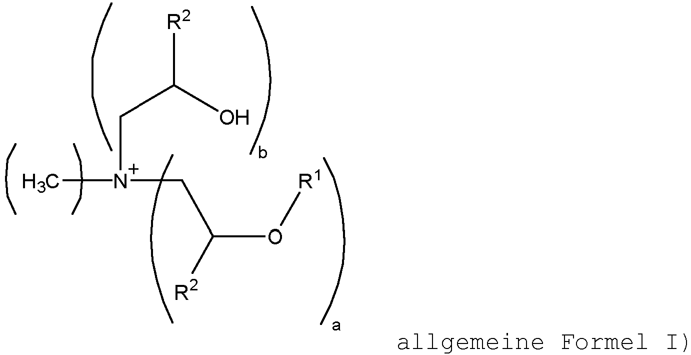 mit
R¹ Acylrest einer Fettsäure,
R² unabhängig voneinander ausgewählt aus Alkylresten mit 1 bis 6 Kohlenstoffatomen und H, insbesondere H,
mit
a = 1,0 bis 2,2, bevorzugt 1,5 bis 2,0, besonders bevorzugt 1,7 bis 1,9, insbesondere 1,8, und
b = 3 - a,
**dadurch gekennzeichnet, dass**
R¹ eine Mischung von Acylresten darstellt, welche Folgendes aufweist:
ein Gewichtsverhältnis von gesättigten C16-Acylresten zu ungesättigten C16-Acylresten größer 2 und
ein Gewichtsverhältnis von ungesättigten C18-Acylresten zu gesättigten C18-Acylresten größer als 10.

2. Zusammensetzung nach Anspruch 1, **dadurch gekennzeichnet, dass** die Mischung von Acylresten einen Gehalt an Acylresten wie folgt dargestellt enthält:
| | |
|---|---|
| C16 | 4 Gew.-% - 7 Gew.-%, |
| C16:1 | 0 Gew.-% - 2 Gew.-%, |
| C18 | 0 Gew.-% - 4 Gew.-%, |
| C18:1 | 55 Gew.-% - 65 Gew.-%, |
| C18:2 | 15 Gew.-% - 25 Gew.-%, |
| C18:3 | 6 Gew.-% - 12 Gew.-%, |
wobei sich die Gewichtsprozente auf die Summe aller R¹ in dem Esterquat der allgemeinen Formel I) beziehen.

3. Zusammensetzung nach Anspruch 1, **dadurch gekennzeichnet, dass** die Mischung von Acylresten einen Gehalt an Acylresten wie folgt dargestellt enthält:
| | |
|---|---|
| C16 | 4 Gew.-% - 7 Gew.-%, |
| C16:1 | 0 Gew.-% - 2 Gew.-%, |
| C18 | 0 Gew.-% - 4 Gew.-%, |
| C18:1 | 14 Gew.-% - 21 Gew.-%, |
| C18:2 | 12 Gew.-% - 25 Gew.-%, |
| C18:3 | 29 Gew.-% - 39 Gew.-%, |
wobei sich die Gewichtsprozente auf die Summe aller R¹ in dem Esterquat der allgemeinen Formel I) beziehen.

4. Zusammensetzung nach Anspruch 1, **dadurch gekennzeichnet, dass** die Mischung von Acylresten einen Gehalt an Acylresten wie folgt dargestellt enthält:
| | |
|---|---|
| C16 | 1 Gew.-% - 8 Gew.-%, |
| C16:1 | 0 Gew.-% - 2 Gew.-%, |
| C18 | 0 Gew.-% - 4 Gew.-%, |
| C18:1 | 40 Gew.-% - 60 Gew.-%, |
| C18:2 | 30 Gew.-% - 40 Gew.-%, |
| C18:3 | 5 Gew.-% - 10 Gew.-%, |
wobei sich die Gewichtsprozente auf die Summe aller R¹ in dem Esterquat der allgemeinen Formel I) beziehen.

5. Zusammensetzung nach einem der Ansprüche 2 bis 4, **dadurch gekennzeichnet, dass** die Summe der konkret benannten Acylreste mindestens 70 Gew.-%, insbesondere mindestens 80 **Gew.-%,** besonders bevorzugt mindestens 90 **Gew.-%,** bezogen auf alle in der Mischung enthaltenen Acylreste, ausmacht.

6. Zusammensetzung nach mindestens einem der vorhergehenden Ansprüche, **dadurch gekennzeichnet, dass** die Mischung von Acylresten eine Iodzahl von 80 bis 170, bevorzugt 95 bis 160, besonders bevorzugt 105 bis 135 cg I/g, aufweist.

7. Zusammensetzung nach mindestens einem der vorhergehenden Ansprüche, zusätzlich enthaltend mindestens ein Gegenanion zu der Verbindung der allgemeinen Formel I) ausgewählt aus der Gruppe umfassend Chlorid, Bromid, Iodid, Alkylsulfat, beispielsweise Methylsulfat, Ethylsulfat, Alkylsulfonat, beispielsweise Methylsulfonat, Triflat, Tosylat, Phosphat, Sulfat, Hydrogensulfat, Lactat, Glycolat, Acetat und Citrat.

8. Zusammensetzung nach mindestens einem der vorhergehenden Ansprüche, **dadurch gekennzeichnet, dass** die Zusammensetzung eine Formulierung, insbesondere eine kosmetische Formulierung, ist.

9. Zusammensetzung nach mindestens einem der vorhergehenden Ansprüche, **dadurch gekennzeichnet, dass** das Esterquat in einer Menge von 0,2 bis 25 Gew.-%, bevorzugt 0,5 bis 15 Gew.-%, insbesondere 1 bis 10 Gew.-%, enthalten ist, wobei sich die Gewichtsprozente auf die Gesamtzusammensetzung beziehen.

10. Zusammensetzung nach mindestens einem der vorhergehenden Ansprüche, **dadurch gekennzeichnet, dass** sie mindestens ein Alkylamidoamin enthält, das bevorzugt in einer Menge von 0,2 Gew.-% bis 25 Gew.-%, bevorzugt 0,5 Gew.-% bis 15 Gew.-%, insbesondere 1,0 Gew.-% bis 10 Gew.-%, enthalten ist, wobei sich die Gewichtsprozente auf die Gesamtzusammensetzung beziehen.

11. Verwendung einer Zusammensetzung nach mindestens einem der vorhergehenden Ansprüche zur kosmetischen Behandlung von Keratinfasern.

12. Verwendung nach Anspruch 11 zur Erhöhung des Glanzes der Faser.

13. Verwendung nach Anspruch 11 zur Erhöhung der Flexibilität, der Kämmbarkeit und/oder der Spannkraft der Faser.

14. Verwendung nach Anspruch 11 zur Reduktion der antistatischen Kräfte zwischen den Fasern.

15. Verwendung nach Anspruch 11 zum Schutz der Fasern vor Hitze.

## Revendications

1. Composition comprenant un ester quat de la formule générale I) 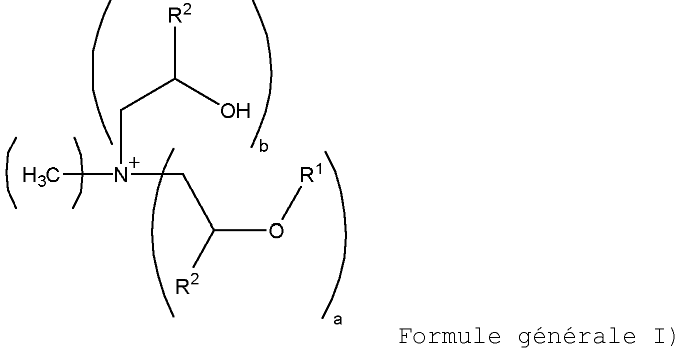 où
R¹ est un radical acyle d'un acide gras,
R² est indépendamment choisi parmi des radicaux alkyle ayant 1 à 6 atomes de carbone et H, en particulier H,
où
a = 1,0 à 2,2, de préférence 1,5 à 2,0, de manière particulièrement préférée 1,7 à 1,9, en particulier 1,8, et
b = 3 - a,
**caractérisée en ce que**
R¹ est un mélange de radicaux acyle qui a
un rapport pondéral des radicaux acyle en C16 saturés sur radicaux acyle en C16 insaturés supérieur à 2 and
un rapport pondéral des radicaux acyle en C18 insaturés sur radicaux acyle en C18 saturés supérieur à 10.

2. Composition selon la revendication 1, **caractérisée en ce que** le mélange de radicaux acyle comprend une teneur en radicaux acyle comme indiquée ci-dessous
| | |
|---|---|
| C16 | 4 % en poids - 7 % en poids, |
| C16:1 | 0 % en poids - 2 % en poids, |
| C18 | 0 % en poids - 4 % en poids, |
| C18:1 | 55 % en poids - 65 % en poids, |
| C18:2 | 15 % en poids - 25 % en poids, |
| C18:3 | 6 % en poids - 12 % en poids, |
où les pourcentages en poids sont basés sur la somme totale de tous les R¹ dans l'ester quat de la formule générale I).

3. Composition selon la revendication 1, **caractérisée en ce que** le mélange de radicaux acyle comprend une teneur en radicaux acyle comme indiquée ci-dessous
| | |
|---|---|
| C16 | 4 % en poids - 7 % en poids, |
| C16:1 | 0 % en poids - 2 % en poids, |
| C18 | 0 % en poids - 4 % en poids, |
| C18:1 | 14 % en poids - 21 % en poids, |
| C18:2 | 12 % en poids - 25 % en poids, |
| C18:3 | 29 % en poids - 39 % en poids, |
où les pourcentages en poids sont basés sur la somme totale de tous les R¹ dans l'ester quat de la formule générale I).

4. Composition selon la revendication 1, **caractérisée en ce que** le mélange de radicaux acyle comprend une teneur en radicaux acyle comme indiquée ci-dessous
| | |
|---|---|
| C16 | 1 % en poids - 8 % en poids, |
| C16:1 | 0 % en poids - 2 % en poids, |
| C18 | 0 % en poids - 4 % en poids, |
| C18:1 | 40 % en poids - 60 % en poids, |
| C18:2 | 30 % en poids - 40 % en poids, |
| C18:3 | 5 % en poids - 10 % en poids, |
où les pourcentages en poids sont basés sur la somme totale de tous les R¹ dans l'ester quat de la formule générale I).

5. Composition selon l'une quelconque des revendications 2 à 4, **caractérisée en ce que** la somme totale des quantités de radicaux acyle spécifiquement cités s'élève à au moins 70 % en poids, en particulier au moins 80 % en poids, de manière particulièrement préférée au moins 90 % en poids, par rapport à tous les radicaux acyle présents dans le mélange.

6. Composition selon au moins l'une des revendications précédentes, **caractérisée en ce que** le mélange de radicaux acyle présente un indice d'iode de 80 à 170, de préférence de 95 à 160, de manière particulièrement préférée de 105 à 135 cg de I/g.

7. Composition selon au moins l'une des revendications précédentes, contenant de plus au moins un contre-anion au composé de formule générale I) choisi dans le groupe comprenant chlorure, bromure, iodure, alkylsulfate, par exemple méthylsulfate, éthylsulfate, alkylsulfonate, par exemple méthylsulfonate, triflate, tosylate, phosphate, sulfate, hydrogénosulfate, lactate, glycolate, acétate et citrate.

8. Composition selon au moins l'une des revendications précédentes, **caractérisée en ce que** la composition est une formulation, en particulier une formulation cosmétique.

9. Composition selon au moins l'une des revendications précédentes, **caractérisée en ce que** l'ester quat est présent en une quantité de 0,2 % à 25 % en poids, de préférence de 0,5 % à 15 % en poids, en particulier de 1 % à 10 % en poids, les pourcentages en poids étant basés sur la composition totale.

10. Composition selon au moins l'une des revendications précédentes, **caractérisée en ce qu'**elle comprend au moins une alkylamidoamine, qui est présente de préférence en une quantité de 0,2 % en poids à 25 % en poids, de préférence de 0,5 % en poids à 15 % en poids, en particulier de 1,0 % en poids à 10 % en poids, les pourcentages en poids étant basés sur la composition totale.

11. Utilisation d'une composition selon au moins l'une des revendications précédentes pour le traitement cosmétique de fibres kératiniques.

12. Utilisation selon la revendication 11 pour augmenter la brillance de la fibre.

13. Utilisation selon la revendication 11 pour augmenter la flexibilité, la peignabilité et/ou l'élasticité de la fibre.

14. Utilisation selon la revendication 11 pour réduire les forces antistatiques entre les fibres.

15. Utilisation selon la revendication 11 pour la protection des fibres contre la chaleur.
